**Europäisches Patentamt**

**European Patent Office**

**Office européen des brevets**

(19)

(11) Publication number : **0 545 951 B1**

## (12) EUROPEAN PATENT SPECIFICATION

(45) Date of publication of patent specification :
**22.06.94 Bulletin 94/25**

(51) Int. Cl.[5] : **C07K 7/08,** C07K 7/10,
C07K 7/06, A61K 39/155

(21) Application number : **91913753.9**

(22) Date of filing : **30.07.91**

(86) International application number :
**PCT/US91/05255**

(87) International publication number :
**WO 92/04375 19.03.92 Gazette 92/07**

(54) **HRSV VACCINE.**

(30) Priority : **31.08.90 US 575892**

(43) Date of publication of application :
**16.06.93 Bulletin 93/24**

(45) Publication of the grant of the patent :
**22.06.94 Bulletin 94/25**

(84) Designated Contracting States :
**AT BE CH DE DK ES FR GB GR IT LI LU NL SE**

(56) References cited :
**WO-A-89/02935**
**Journal of General Virology, vol. 71, 1990 (GB),**
**G.E. Scopes et al., pp. 53-59**
**Journal of Virology, vol. 65, no. 7, July 1991,**
**Am. Soc. for Microbiol., M.E. Levely et al., pp.**
**3789-3796**

(73) Proprietor : **THE UPJOHN COMPANY**
**301 Henrietta Street**
**Kalamazoo, Michigan 49001 (US)**

(72) Inventor : **NICHOLAS, Judith, A.**
**4120 C Avenue East**
**Kalamazoo, MI 49004 (US)**

(74) Representative : **Perry, Robert Edward (GB) et**
**al**
**Gill Jennings & Every**
**Broadgate House**
**7 Eldon Street**
**GB-London EC2M 7LH (GB)**

## Description

FIELD OF THE INVENTION

The present invention relates to an immunogenic peptide useful in a vaccine for protecting humans against Human Respiratory Syncytial Virus (HRSV) and a method of protecting humans against HRSV.

BACKGROUND OF THE INVENTION

HRSV was discovered in 1956 and is found worldwide. It causes upper and lower respiratory tract disease particularly in infants and young children. About 30 percent of hospitalized young children with acute respiratory disease have HRSV infection. In older children and adults the disease is milder.

Infections with HRSV are referable to all segments of the respiratory tract, are usually associated with fever, cough, runny nose, and fatigue, and are clinically diagnosed as bronchitis, bronchiolitis, pneumonia, croup, or viral infection. In older children and adults the virus is generally limited to replication in the upper respiratory tract. Infants may be more severely involved when the virus extends into the lungs. Lung damage can be permanent.

Primary infection with HRSV occurs early in life, usually before two years of age. Among children, illness caused by this virus tends to occur at least once each year in rather sharply defined outbreaks of several months duration. Epidemics are sharply circumscribed, generally for three to five months. In family studies, children in early school years frequently introduce the virus into the home, infecting younger members of the family more severely than other family members. The clinical consequence of infection is most severe on first and second experience and becomes milder in older individuals who are immunologically experienced.

Secondary effects of HRSV can range from unapparent infection to severe pneumonia and death. Inflammation of the respiratory track is responsible for most symptoms. Complete recovery in most cases occurs in one to three weeks with the production of antibody which appears to persist throughout life. In the United States about 30% of one year old infants and 95% of five year old children have circulating HRSV antibody. Reinfections in older infants, children, and adults with antibody are mostly mild upper respiratory illnesses in the form of colds.

Although low yields of virus in cell culture have hindered HRSV research, the virus has been well studied. HRSV is a paramyxovirus containing a single negative strand of RNA which is transcribed into 10 monocistronic messengers. The messengers have been isolated and translated in vitro. The products have been characterized by gel electrophoresis, peptide mapping and immunoprecipitation as being similar to structural proteins isolated from virions. The structural proteins include: a major nucleocapsid protein (N; MW ca. 42,000); a nucleocapsid phosphoprotein (P; MW ca. 34,000); a large nucleocapsid protein (L; MW ca. 200,000); an envelope matrix protein (M; MW ca. 26,000); a second matrix protein (MW ca. 22,000); and two envelope glycoproteins, the fusion glycoprotein (F; MW ca. 68,000 to 70,000) and a second, methionine poor glycoprotein (G; MW ca. 84,000 to 90,000). In addition, another protein, referred to as the 1A protein is about a 9,500 dalton protein and found in the membrane fraction of purified HRSV and in the membrane of virus-infected cells. In addition, other small proteins are known to be present in infected cells, Collins, P.L., et al., J. Virol. 49:572 - 578 (1984) and references cited therein. Morgan, L. A., et al., J. gen. Virol. 68:2781-2788 (1987) refers to strain variation and HRSV.

There are no effective vaccines to combat HRSV currently available. Multiple attempts have been made to obtain an effective vaccine against HRSV. Friedewald, et al., J. Am. Med. Asso., 204:690-694 (20 May 1968) describe the propagation of HRSV in bovine embryonic kidney tissue culture. Virus grown at 34°C or 28°C did not decrease in infectivity or virulence. HRSV grown at 26°C, while associated with a decrease in infectivity for adults, could not be considered for use in prevention of infection in adults since the virus had limited infectivity and was poorly immunogenic. Kim, et al., Pediatrics, 48:745-755 (November 1971) disclose that inactivated HRSV vaccine prepared from virus grown at 26°C stimulated the development of high levels of serum antibody in infants and children from six months to thirteen years in age but did not prevent infection. McIntosh, et al., Pediatr. Res., 8:689-696 (1974) discuss two experimental live HRSV vaccines, one prepared from virus grown at 26°C. and the other, prepared from a temperature sensitive mutant which grew well at 32°C and not at all at 37°C. or higher. The first vaccine was unsatisfactory as it did not protect against infection when the interval between vaccination and challenge was greater than four months. The second vaccine was also unsatisfactory in that it apparently lost its temperature sensitivity in some vaccinees. Craighead, J. Infect. Dis., 131:749-753 (June 1975) discusses tests conducted in 1966 wherein several groups of investigators tested in infants and young children a formaldehyde-treated, alum-precipitated virus grown in tissue culture. Upon subsequent exposure to wild virus the vaccine recipients exhibited an accentuated pattern of respiratory tract disease. Craighead concludes that immunization with formaldehyde treated virus enhanced the severity of the disease. Wright, et al., J. Pediatr., 88:931-936 (June 1976) describe the evaluation in infants of a temperature sensitive live attenuated HRSV vaccine. While this vaccine when administered at a dosage level sufficiently high to infect all seronegative infants caused mild upper respiratory illness, lowering the dose did not achieve an acceptable level of infectivity. The virus was also genetically unstable as there was evidence of loss of temperature sensitivity in one vaccinee. There was no evidence for potentiation of natural illness with this vaccine and reinfection occurred among vaccinees.

U.S. Patent No. 4,517,304 discloses a method for producing immunogenically active HRSV proteins upon the cell membranes of susceptible cells grown in culture. These cells are then injected into a host to elicit an immune response.

Subunit vaccines involving HRSV proteins have been proposed. Such vaccines can consist of HRSV proteins administered to a patient in order to provide an antigen useful as an antibody target. Alternatively, vaccinia virus expression systems can be used to provide HRSV proteins to serve as antigen targets for a protective immune response. The vaccinia virus expression system is known to separately express several HRSV proteins including the G and F glycoproteins of HRSV. Ball, et al., P.N.A.S. USA 83:246-250 (1986) and Olmsted, P.N.A.S. USA 83:7462-7466 (1986). These two glycoproteins were demonstrated to induce immunoprotection in mammals against a live HRSV virus challenge: Stott, et al., Journal of Virology 67:607-613 (1986); Walsh, et al., Journal of Infectious Diseases, 1198-1204 (1987); Wertz, et al., Journal of Virology, 293-301 (1987); Elango, et al.; and Olmsted, R.A., P.N.A.S. USA 83:246-250 (1986).

In each case, attempts made to provide a vaccine capable of conferring protection against infection have involved providing antigenic targets which the patient can be exposed to and, accordingly, develop an antibody based immune response to subsequent infection. The immune system functions to recognize and eliminate "foreign invaders" including those associated with bacterial or viral infection such as HRSV. The lymphocytes are elemental constituents of the immune system. These cells can be classified as B-lymphocytes (B-cells) or T-lymphocytes (T-cells). B-cells and some T-cells are involved in the production of antibodies, molecules that recognize and bind to specific patterns displayed by invading infective agents. The specific patterns that are recognized by antibodies are called epitopes, antigenic determinants or antigenic sites. Molecules which display epitopes are called antigens. An epitope is typically part of a folded chain found at the surface of a protein molecule. B-cells and their progeny cells can secrete antibodies. T-cells do not secrete antibodies but do nonetheless recognize epitopes.

One activity an antigen may induce is the proliferation of T-cells. Upon being exposed to discrete epitopes, the T-cells specific against that epitope will replicate at a higher rate than unstimulated T-cells. The proliferating T-cells are specific against the epitope and will function in the immune response against that epitope. This is, whatever role a particular T-cell may play in an immunogenic response against an epitope, such a response will be enhanced by exposure to an antigen which causes proliferation, i.e., replication of that T-cell.

There are several different kinds of T-cells. Some T-cells suppress B-cell activity. Other T-cells, helper T-cells, help B-cells become stimulated. A helper T-cell which exhibits a proliferative response will stimulate B-cells to make antibodies against the same or nearby epitopes that stimulated the helper T-cell. Killer or cytotoxic T-cells (CTL) are not involved in antibody based defenses against infection. Rather, CTL recognize, attack and kill other cells such as cancer cells, transplanted tissue or cells infected with a virus. The molecular mechanisms which control the immune response are not fully understood and much remains to be elucidated about the processes by which peptidic epitopes elicit defined immune responses.

There has been some work reported that demonstrates some proteins stimulate helper T-cell activity. Openshaw, P. J. et al., J. Gen. Virol. 69:305-312 (1988) disclose protein specificities of helper T-cells to HRSV. Fusion protein of HRSV was found to induce a stronger helper T-cell response than the G glycoprotein of HRSV.

The present invention relates to a peptide fragment which provides improved means to protect a human from HRSV infection. By stimulating T-cell activity, a human exposed to the peptide fragments of the present invention is protected from HRSV infection. Protection conferred by stimulated T-cell activity is activated faster than humoral protection when viral challenge occurs. In addition, protection derived from stimulated CTL activity lasts longer than humoral protection.

The present invention provides a peptide which is useful in a vaccine that can confer protection against HRSV infection. The peptide of the present invention is derived from amino acid residues 328-355 of the F glycoprotein from HRSV and fragments thereof. A vaccine comprising a peptide according to the present invention stimulates proliferation of T-cells that are active in the immunologic response to HRSV infection.

INFORMATION DISCLOSURE

PCT Patent Application Number PCT/US86/02756 discloses vaccines for conferring resistance to HRSV infection comprising native structural viral proteins including the F glycoprotein. Production of the viral proteins by recombinant expression of the DNA encoding them is also disclosed. The protein is purified and formulated into a vaccine.

PCT Patent Application Number PCT/US88/03784 discloses DNA compositions encoding novel chimeric glycoproteins which are useful for preparing virus specific immune responses against HRSV. The chimeric glycoproteins described are a combination of portions from both the F and G glycoproteins of HRSV. The chimeric glycoproteins produced are useful to make vaccines which confer resistance to HRSV infection.

PCT Patent Application Number PCT/US88/03623 discloses peptides derived from the residues 45 to 64 of the 1A protein of HRSV. The peptides are useful as vaccines against human and bovine HRSV infection. The peptides stimulate helper T-cell function whereby an antibody response is enhanced. The peptides disclosed are delivered as a vaccine.

Openshaw, P. J., et al., J. Virol. 64(4):1683-1689, April 1990, discloses recombinant vaccinia viruses containing HRSV 22-K (Vac22K) protein used to infect target cells for cultured polyclonal CTL. The data indicates the 22-K protein as a major target antigen from an HRSV-specific CTL from $H-2^d$ mice primed by HRSV infection. Inoculation of mice with Vac22K induced minimal CTL activity for HRSV.

Nicholas, J., et al., J. Virol. 64(9):4232-4241, September 1990, disclose mouse HRSV specific CTL recognize HRSV 22K protein as the major CTL antigen.

Olmsted, R. A., et al., Proc. Natl. Acad. Sci. USA 83:7462-7466, 1986, disclose a cDNA clone representing the mRNA coding sequence for the fusion glycoprotein (F) virus which was constructed and inserted into the thymidine kinase gene of vaccinia virus under the control of a vaccinia virus promoter. The resulting recombinant vaccinia virus, vaccinia F, expressed the $F_1$ and $F_2$ cleavage products of the F glycoprotein in cell cultures. $F_1$ and $F_2$ were indistinguishable from their authentic HRSV counterparts. Cotton rats infected intradermally with vaccinia F developed at high titer of serum F-specific antibodies, which neutralized infectivity of HRSV.

Walsh, et al., J. Infect. Dis., 155:1198-1204, 1987, disclose that animals immunized with purified attachment protein (G) or fusion protein (F) of HRSV developed complete pulmonary resistance, but only partial nasal resistance, to challenge with HRSV. The viral glycoproteins were purified and introduced into the animals intraperitoneally.

Wertz, et al., J, Virol. 61:293-301, 1987, disclose a vaccinia virus recombinants that were constructed which contained full-length cDNA copies of the fusion (F) protein gene of HRSV. Inoculation of rabbits with the recombinant vector expressing F resulted in production of antiserum specific for the HRSV F protein. This antiserum neutralized viral infectivity and was capable of preventing fusion in HRSV infected cells.

Bangham, C. R. M. and Askonas, B. A., J. gen. Virol 67:623-629, 1986, disclose that CTL raised against HRSV in the mouse are specific to other strains of immunizing virus or cross-reactive between virus strains.

Pemberton, R. M., et al., J. gen. Virol. 68:2177-2182, 1987, disclose that the F protein is an important T antigen in CTL specificity for HRSV proteins.

## SUMMARY OF THE INVENTION

The present invention relates to a use of a fragment of the HRSV F glycoprotein to protect against infection by HRSV. In addition, the present invention provides the nucleotide sequence encoding the amino acid sequence of the immunogenically active fragment of the F glycoprotein. The present invention relates to a method of using the immunogenic fragment of the F glycoprotein to confer protection against HRSV infection.

## DETAILED DESCRIPTION OF THE INVENTION

The present invention provides a peptide having the amino acid sequence derived from amino acid residues 328-355 by the F glycoprotein of HRSV and fragments thereof. As used herein, "Ffrag" refers to the peptide having an amino acid sequence of (328-355) Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala; and fragments thereof. Ffrag can be made using a peptide synthesize- or using recombinant DNA technology. A vaccine which is effective in conferring protection against HRSV infection can be prepared using Ffragby one having ordinary skill in the art.

The mechanism by which Ffrag confers protection is through stimulation of a T-cell proliferative response. Accordingly, T-cells coming into contact with Ffrag are stimulated to replicate. The progeny are therefore available in greater numbers to recognize and respond to the Ffrag epitope. The Ffrag epitope is found in the F glycoprotein of HRSV. Accordingly, the T-cell activity stimulated by Ffrag is useful to respond to HRSV challenge and thereby confers protection against HRSV infection.

The stimulation of T-cell activity by Ffrag is demonstrated by studying T-cell response to exposure to HRSV and fragments of F glycoprotein including Ffrag. The response of human T-cells derived from eight normal adult donors upon restimulation with HRSV was evaluated. Although the level of responsiveness varied between donors, seven out of the eight individuals mounted a significant T-cell proliferative response to restimulation with HRSV.

Through the use of a nested set of overlapping truncated peptides spanning the extracellular and cytoplasmic domains of the F glycoprotein of HRSV, one immunodominant T-cell stimulating epitope was identified which is found within residues 328-355. Monoclonal antibody (MAb) blocking studies revealed that DR2 was the MHC class II restriction element which presented the immunodominant Ffrag epitope in three individuals. The present invention relates to peptides modelled after the immunodominant T-cell epitope located on the F glycoprotein that is presented by the MHC class II DR2 determinant following natural HRSV infection in humans.

The first set of experiments evaluated the T-cell proliferative response of human cells after restimulation with HRSV. This data was used to identify high responder and intermediate responder donors. In the second set of experiments, restimulation of the identified responders with the nested set of peptides was used to identify the immunodominant epitope of the F glycoprotein.

Peripheral blood mononuclear cells (PBMC) were derived from eight normal adult donors over the course of a year via leukapheresis, purified on ficoll (Pharmacia, Piscataway, NJ), aliquoted and stored frozen in 10% DMSO in liquid nitrogen until use. All donors in the panel were presumed to have encountered multiple natural infections with HRSV during their lifetimes. Recent HRSV infection was confirmed in one individual from whom HRSV was isolated during hospitalization a few months prior to PBMC donation. PBMC from each donor were restimulated *in vitro* with HRSV and the resulting proliferative response was compared to the response obtained when cultures were restimulated medium alone. HRSV (A2 strain) was grown in HEp2 cells in Eagle's MEM (Whittaker Bioproducts, Walkersville, MD) containing 4% fetal bovine serum (FBS, Gibco, Grand Island, NY) and titered by plaque assay under 5% agarose containing 3% FBS. For restimulation *in vitro,* PBMC were cultured at $4 \times 10^5$ cells per well in microtiter in MLR medium containing a final concentration of 10% human AB serum (Hazleton, Denver, PA). To each well was added an equal volume of medium containing dilutions of the HRS virus. Virus was added at a multiplicity of infection of 0.1 to 1.0 viral particles per cell. Cultures were incubated for five days then cells were pulse labeled for 6 hr with 1 μg/well of $^3$H-thymidine (ICN Pharmaceuticals, Irvine, CA). Cells were then collected and T-cell responses were expressed as CPM $^3$H-thymidine incorporation or were calculated as stimulation index (SI) +/- std. dev. where SI=mean cpm in the presence of antigen/mean cpm in the presence of medium alone. Measure of thymidine incorporation allows for the relative quantitation of DNA production, a standard measure of cell replication.

PBMC responses to restimulation with HRSV varied between donors and fell into three categories: high responders (3), intermediate responders (5) and one low responder. The HRSV specific proliferative responses are consistent with the stimulation of T-cells. Thus, hereafter, the population of cells responding to restimulation with either antigen or individual epitopes thereof are referred to as T-cells. The data presented herein reveal that HRSV stimulated proliferation in a panel of human peripheral blood T lymphocytes obtained from healthy, adult donors.

The next set of experiments were designed to determine which epitopes on the F glycoprotein were recognized by the responding T-cells and stimulating cell replication of the T-cells. A series of fifty-two overlapping peptides of the F glycoprotein were synthesized and purified. Each truncated peptide is a fragment of Ffrag is referred to as Ffrag (xxx-yyy) wherein (xxx-yyy) refers to the first and last residues of the peptide. Peptides were synthesized by solid phase methodology on an Applied Biosystems Inc. (ABI) 430A peptide synthesizer using ABI's Small Scale Rapid Cycles (SSRC) on a 0.1 mmole scale. SSRC utilizes abbreviated single couple cycles with standard Boc chemistry. The t-Boc-L-amino acids used (1 mmole) were supplied by ABI with standard side-chain protecting groups. The completed peptides were removed from the supporting PAM (phenylacetamidomethyl) resin, concurrently with the side-chain protecting groups, by a standard HF procedure using appropriate cation scavengers (10% v/v anisole, p-cresol plus p-thiocresol, 1,4-butanedithiol plus anisole or DMS plus anisole) depending on the amino acid sequence of the peptide.

The crude peptides, after HF cleavage, were purified by preparative reverse phase chromatography on a Phenomenex C-18 Column (250 x 22.5 mm) using water acetonitrile gradients, each phase containing 0.1 % TFA. The pure fractions (as determined by analytical HPLC) were pooled, acetonitrile evaporated and the aqueous solution lyophilized. All compounds were analyzed by fast atom bombardment mass spectroscopy and gave the anticipated $(M+H)^+$.

As in the first set of experiments, PBMC were derived from normal adult donors via leukapheresis, purified on ficoll (Pharmacia, Piscataway, NJ), aliquoted and stored frozen in 10% DMSO in liquid nitrogen until use. For these studies, two representative donors, one intermediate responder and one high responder were chosen. For restimulation *in vitro* with the peptides, PBMC were cultured at $4 \times 10^5$ cells per well in microtiter in MLR medium containing a final concentration of 10% human AB serum (Hazleton, Denver, PA). To each well was added an equal volume of medium containing dilutions of the appropriate synthetic peptide or HRSV. HRSV was added at a multiplicity of infection of 0.1 to 1.0 viral particle per cell. Peptides were added in media at a concentration of 0.01-100 µg/ml. To each well .2 ml of this media was added. Cultures were incubated for five days then cells were pulse labeled for 6 hr with 1 µg/well of $^3$H-thymidine (ICN Pharmaceuticals, Irvine, CA). Cells were then collected and T-cell responses were expressed as CPM $^3$H-thymidine incorporation or were calculated as stimulation index (SI) +/-std. dev. where SI=mean cpm in the presence of antigen/mean cpm in the presence of medium alone.

T-cells from intermediate response donor were restimulated with the complete set of truncated F peptides. Only one peptide, Ffrag$_{(338-355)}$, induced significant, reproducible restimulation of this donor's T-cells. T-cells from high response donor consistently responded to restimulation with Ffrag$_{(328-342)}$ or Ffrag$_{(338-355)}$. Most strikingly, T-cells from both donors responded strongly to restimulation with Ffrag$_{(338-355)}$. Therefore, to further characterize this response, the following MAb blocking study was done.

Purified MAbs anti-HLA-DR, Leu 3a and Leu 6 with specificities for monomorphic human class II histocompatibility determinants, human CD4 and thymocyte CD1 antigen, respectively, were purchased from Becton Dickinson, Mountain View, CA. All antibodies were dialyzed against 1000 volumes of PBS to remove sodium azide. For blocking studies, a final concentration of 0.5 1g/ml of purified MAb was included in proliferation assays at the time of initiation of peptide restimulation. Results are expressed as percent inhibition of peptide induced proliferation where

$$\% \text{ Inhibition} = 1 - \frac{(\text{CPM w/Peptide} + \text{MAb}) - (\text{CPM w/MAb})}{(\text{CPM w/Peptide}) - (\text{CPM w/media})} \times 100$$

Comparison of the HLA profiles of two donors revealed that both individuals shared DR2 and DQw1 MHC class II determinants. T-cells from both donors were restimulated with Ffrag$_{(338-355)}$ in the presence of purified MAb directed against DR MHC class II determinants, CD4 or thymocyte CD1 antigen to determine if Ffrag$_{(338-355)}$ was being presented in association with DR2 or DQw1. The latter MAb to CD1 served as a non-specific blocking control. The ability of these MAbs to inhibit Ffrag$_{(338-355)}$-induced proliferation in T-cells from the intermediate response donor was shown. The results showed that anti-DR antibody significantly blocked Ffrag$_{(338-355)}$-induced proliferation and anti-CD4 antibody also moderately blocked Ffrag$_{(338-355)}$-induced proliferation. A slight blocking effect was seen with anti-CD1 at the higher peptide concentration but no blocking was observed at the lower peptide concentration. Similarly, when T-cells from the high response donor were restimulated with Ffrag$_{(338-355)}$, peptide specific proliferation was efficiently blocked by either anti-DR or anti-CD4 antibody. These data suggested that Ffrag$_{(338-355)}$ was being presented in association with DR2 determinants in these two individuals and confirmed that the responding cell population was composed of CD4$^+$ T-cells. Additional studies demonstrated that peptide-induced proliferation of the high response donor T-cells by Ffrag$_{(328-342)}$ was also efficiently blocked by anti-DR or anti-CD4 antibody. Experiments using PBMC derived from a third donor confirmed the results of those performed on the high response donor. The third donor displayed stimulated T-cell proliferative activity when exposed to Ffrag$_{(328-342)}$ and Ffrag$_{(338-355)}$.

Thus, using the nested set of overlapping peptides spanning the extracellular and cytoplasmic domains of the F glycoprotein the T-cell stimulating epitopes in a high responder and an intermediate responder to HRSV were mapped. T-cells from both donors were strongly stimulated to proliferate in the presence of the peptide spanning residues 338 to 355. Additionally, the peptide spanning residues 328 to 342 stimulated T-cells from the high response donor. Tissue typing of EBV-transformed B lymphoblastoid cells from these two donors revealed that they shared two alleles, DR2 and DQw1, of the class II MHC gene complex, either of which could be involved in presentation of the 338 to 355 peptide. Monoclonal antibody directed against non-polymorphic DR determinants blocked the proliferative response to this peptide in both donors, suggesting that DR2 was the antigen presenting element. These data indicate that the peptide spanning residues 328 to 355 encompass all or part of a major, immunodominant T-cell epitope of the F glycoprotein for humans expressing DR2.

The present invention provides a peptide having an amino acid sequence modelled after the 328 to 355 residues of the HRSV F glycoprotein or subunits thereof and vaccines comprising such peptides. Peptides according to the present invention include:

(328-355)    Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(329-355)    Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(330-355)    Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(331-355)    Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(332-355)    Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(333-355)    Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(334-355)    Leu-Thr-Arg-Thr-Asp-Arg-Gly-Trp-Tyr-Cys-Asp-Asn-Ala-Gly-Ser-Val-Ser-Phe-Phe-Pro-Gln-Ala;

(335-355)    Thr-Arg-Thr-Asp-Arg-Gly-Trp-Tyr-Cys-Asp-Asn-Ala-Gly-Ser-Val-Ser-Phe-Phe-

Pro-Gln-Ala;

(336-355)      Arg-Thr-Asp-Arg-Gly-Trp-Tyr-Cys-Asp-Asn-Ala-Gly-Ser-Val-Ser-Phe-Phe-Pro-Gln-Ala;

(337-355)      Thr-Asp-Arg-Gly-Trp-Tyr-Cys-Asp-Asn-Ala-Gly-Ser-Val-Ser-Phe-Phe-Pro-Gln-Ala;

(338-355)      Asp-Arg-Gly-Trp-Tyr-Cys-Asp-Asn-Ala-Gly-Ser-Val-Ser-Phe-Phe-Pro-Gln-Ala;

(328-354)      Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(329-354)      Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(330-354)      Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(331-354)      Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(332-354)      Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(333-354)      Cys-Leu-Thr-Arg-Thr-Asp-Arg-Gly-Trp-Tyr-Cys-Asp-Asn-Ala-Gly-Ser-Val-Ser-Phe-Phe-Pro-Gln;

(334-354)      Leu-Thr-Arg-Thr-Asp-Arg-Gly-Trp-Tyr-Cys-Asp-Asn-Ala-Gly-Ser-Val-Ser-Phe-Phe-Pro-Gln;

(335-354)      Thr-Arg-Thr-Asp-Arg-Gly-Trp-Tyr-Cys-Asp-Asn-Ala-Gly-Ser-Val-Ser-Phe-Phe-Pro-Gln;

(336-354)      Arg-Thr-Asp-Arg-Gly-Trp-Tyr-Cys-Asp-Asn-Ala-Gly-Ser-Val-Ser-Phe-Phe-Pro-Gln;

(337-354)      Thr-Asp-Arg-Gly-Trp-Tyr-Cys-Asp-Asn-Ala-Gly-Ser-Val-Ser-Phe-Phe-Pro-Gln;

(338-354)      Asp-Arg-Gly-Trp-Tyr-Cys-Asp-Asn-Ala-Gly-Ser-Val-Ser-Phe-Phe-Pro-Gln;

(328-353)      Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro;

(329-353)      Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro;

(330-353)      Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro;

(331-353)      Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro;

(332-353)      Ile-Cys-Leu-Thr-Arg-Thr-Asp-Arg-Gly-Trp-Tyr-Cys-Asp-Asn-Ala-Gly-Ser-Val-Ser-Phe-Phe-Pro;

(333-353)      Cys-Leu-Thr-Arg-Thr-Asp-Arg-Gly-Trp-Tyr-Cys-Asp-Asn-Ala-Gly-Ser-Val-Ser-

Phe-Phe-Pro;

(334-353)     Leu-Thr-Arg-Thr-Asp-Arg-Gly-Trp-Tyr-Cys-Asp-Asn-Ala-Gly-Ser-Val-Ser-Phe-Phe-Pro;

(335-353)     Thr-Arg-Thr-Asp-Arg-Gly-Trp-Tyr-Cys-Asp-Asn-Ala-Gly-Ser-Val-Ser-Phe-Phe-Pro;

(336-353)     Arg-Thr-Asp-Arg-Gly-Trp-Tyr-Cys-Asp-Asn-Ala-Gly-Ser-Val-Ser-Phe-Phe-Pro;

(337-353)     Thr-Asp-Arg-Gly-Trp-Tyr-Cys-Asp-Asn-Ala-Gly-Ser-Val-Ser-Phe-Phe-Pro;

(338-353)     Asp-Arg-Gly-Trp-Tyr-Cys-Asp-Asn-Ala-Gly-Ser-Val-Ser-Phe-Phe-Pro;

(328-352)     Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe;

(329-352)     Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe;

(330-352)     Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe;

(331-352)     Asn-Ile-Cys-Leu-Thr-Arg-Thr-Asp-Arg-Gly-Trp-Tyr-Cys-Asp-Asn-Ala-Gly-Ser-Val-Ser-Phe-Phe;

(332-352)     Ile-Cys-Leu-Thr-Arg-Thr-Asp-Arg-Gly-Trp-Tyr-Cys-Asp-Asn-Ala-Gly-Ser-Val-Ser-Phe-Phe;

(333-352)     Cys-Leu-Thr-Arg-Thr-Asp-Arg-Gly-Trp-Tyr-Cys-Asp-Asn-Ala-Gly-Ser-Val-Ser-Phe-Phe;

(334-352)     Leu-Thr-Arg-Thr-Asp-Arg-Gly-Trp-Tyr-Cys-Asp-Asn-Ala-Gly-Ser-Val-Ser-Phe-Phe;

(335-352)     Thr-Arg-Thr-Asp-Arg-Gly-Trp-Tyr-Cys-Asp-Asn-Ala-Gly-Ser-Val-Ser-Phe-Phe;

(336-352)     Arg-Thr-Asp-Arg-Gly-Trp-Tyr-Cys-Asp-Asn-Ala-Gly-Ser-Val-Ser-Phe-Phe;

(337-352)     Thr-Asp-Arg-Gly-Trp-Tyr-Cys-Asp-Asn-Ala-Gly-Ser-Val-Ser-Phe-Phe;

(338-352)     Asp-Arg-Gly-Trp-Tyr-Cys-Asp-Asn-Ala-Gly-Ser-Val-Ser-Phe-Phe;

(328-351)     Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe;

(329-351)     Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe;

(330-351)     Ser-Asn-Ile-Cys-Leu-Thr-Arg-Thr-Asp-Arg-Gly-Trp-Tyr-Cys-Asp-Asn-Ala-Gly-Ser-Val-Ser-Phe;

(331-351)     Asn-Ile-Cys-Leu-Thr-Arg-Thr-Asp-Arg-Gly-Trp-Tyr-Cys-Asp-Asn-Ala-Gly-Ser-Val-Ser-Phe;

(332-351)     Ile-Cys-Leu-Thr-Arg-Thr-Asp-Arg-Gly-Trp-Tyr-Cys-Asp-Asn-Ala-Gly-Ser-Val-Ser-

Phe;

(333-351)    Cys-Leu-Thr-Arg-Thr-Asp-Arg-Gly-Trp-Tyr-Cys-Asp-Asn-Ala-Gly-Ser-Val-Ser-Phe;

(334-351)    Leu-Thr-Arg-Thr-Asp-Arg-Gly-Trp-Tyr-Cys-Asp-Asn-Ala-Gly-Ser-Val-Ser-Phe;

(335-351)    Thr-Arg-Thr-Asp-Arg-Gly-Trp-Tyr-Cys-Asp-Asn-Ala-Gly-Ser-Val-Ser-Phe;

(336-351)    Arg-Thr-Asp-Arg-Gly-Trp-Tyr-Cys-Asp-Asn-Ala-Gly-Ser-Val-Ser-Phe;

(337-351)    Thr-Asp-Arg-Gly-Trp-Tyr-Cys-Asp-Asn-Ala-Gly-Ser-Val-Ser-Phe;

(338-351)    Asp-Arg-Gly-Trp-Tyr-Cys-Asp-Asn-Ala-Gly-Ser-Val-Ser-Phe;

(328-350)    Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser;

(329-350)    Gly-Ser-Asn-Ile-Cys-Leu-Thr-Arg-Thr-Asp-Arg-Gly-Trp-Tyr-Cys-Asp-Asn-Ala-Gly-Ser-Val-Ser;

(330-350)    Ser-Asn-Ile-Cys-Leu-Thr-Arg-Thr-Asp-Arg-Gly-Trp-Tyr-Cys-Asp-Asn-Ala-Gly-Ser-Val-Ser;

(331-350)    Asn-Ile-Cys-Leu-Thr-Arg-Thr-Asp-Arg-Gly-Trp-Tyr-Cys-Asp-Asn-Ala-Gly-Ser-Val-Ser;

(332-350)    Ile-Cys-Leu-Thr-Arg-Thr-Asp-Arg-Gly-Trp-Tyr-Cys-Asp-Asn-Ala-Gly-Ser-Val-Ser;

(333-350)    Cys-Leu-Thr-Arg-Thr-Asp-Arg-Gly-Trp-Tyr-Cys-Asp-Asn-Ala-Gly-Ser-Val-Ser;

(334-350)    Leu-Thr-Arg-Thr-Asp-Arg-Gly-Trp-Tyr-Cys-Asp-Asn-Ala-Gly-Ser-Val-Ser;

(335-350)    Thr-Arg-Thr-Asp-Arg-Gly-Trp-Tyr-Cys-Asp-Asn-Ala-Gly-Ser-Val-Ser;

(336-350)    Arg-Thr-Asp-Arg-Gly-Trp-Tyr-Cys-Asp-Asn-Ala-Gly-Ser-Val-Ser;

(337-350)    Thr-Asp-Arg-Gly-Trp-Tyr-Cys-Asp-Asn-Ala-Gly-Ser-Val-Ser;

(338-350)    Asp-Arg-Gly-Trp-Tyr-Cys-Asp-Asn-Ala-Gly-Ser-Val-Ser;

(328-349)    Glu-Gly-Ser-Asn-Ile-Cys-Leu-Thr-Arg-Thr-Asp-Arg-Gly-Trp-Tyr-Cys-Asp-Asn-Ala-Gly-Ser-Val;

(329-349)    Gly-Ser-Asn-Ile-Cys-Leu-Thr-Arg-Thr-Asp-Arg-Gly-Trp-Tyr-Cys-Asp-Asn-Ala-Gly-Ser-Val;

(330-349)    Ser-Asn-Ile-Cys-Leu-Thr-Arg-Thr-Asp-Arg-Gly-Trp-Tyr-Cys-Asp-Asn-Ala-Gly-Ser-Val;

(331-349)    Asn-Ile-Cys-Leu-Thr-Arg-Thr-Asp-Arg-Gly-Trp-Tyr-Cys-Asp-Asn-Ala-Gly-Ser-Val;

(332-349)    Ile-Cys-Leu-Thr-Arg-Thr-Asp-Arg-Gly-Trp-Tyr-Cys-Asp-Asn-Ala-Gly-Ser-Val;

(333-349)    Cys-Leu-Thr-Arg-Thr-Asp-Arg-Gly-Trp-Tyr-Cys-Asp-Asn-Ala-Gly-Ser-Val;

(334-349)    Leu-Thr-Arg-Thr-Asp-Arg-Gly-Trp-Tyr-Cys-Asp-Asn-Ala-Gly-Ser-Val;

(335-349)    Thr-Arg-Thr-Asp-Arg-Gly-Trp-Tyr-Cys-Asp-Asn-Ala-Gly-Ser-Val;

(336-349)    Arg-Thr-Asp-Arg-Gly-Trp-Tyr-Cys-Asp-Asn-Ala-Gly-Ser-Val;

(337-349)    Thr-Asp-Arg-Gly-Trp-Tyr-Cys-Asp-Asn-Ala-Gly-Ser-Val;

(338-349)     Asp-Arg-Gly-Trp-Tyr-Cys-Asp-Asn-Ala-Gly-Ser-Val;

(328-348)     Glu-Gly-Ser-Asn-Ile-Cys-Leu-Thr-Arg-Thr-Asp-Arg-Gly-Trp-Tyr-Cys-Asp-Asn-Ala-Gly-Ser;

(329-348)     Gly-Ser-Asn-Ile-Cys-Leu-Thr-Arg-Thr-Asp-Arg-Gly-Trp-Tyr-Cys-Asp-Asn-Ala-Gly-Ser;

(330-348)     Ser-Asn-Ile-Cys-Leu-Thr-Arg-Thr-Asp-Arg-Gly-Trp-Tyr-Cys-Asp-Asn-Ala-Gly-Ser;

(331-348)     Asn-Ile-Cys-Leu-Thr-Arg-Thr-Asp-Arg-Gly-Trp-Tyr-Cys-Asp-Asn-Ala-Gly-Ser;

(332-348)     Ile-Cys-Leu-Thr-Arg-Thr-Asp-Arg-Gly-Trp-Tyr-Cys-Asp-Asn-Ala-Gly-Ser;

(333-348)     Cys-Leu-Thr-Arg-Thr-Asp-Arg-Gly-Trp-Tyr-Cys-Asp-Asn-Ala-Gly-Ser;

(334-348)     Leu-Thr-Arg-Thr-Asp-Arg-Gly-Trp-Tyr-Cys-Asp-Asn-Ala-Gly-Ser;

(335-348)     Thr-Arg-Thr-Asp-Arg-Gly-Trp-Tyr-Cys-Asp-Asn-Ala-Gly-Ser;

(336-348)     Arg-Thr-Asp-Arg-Gly-Trp-Tyr-Cys-Asp-Asn-Ala-Gly-Ser;

(337-348)     Thr-Asp-Arg-Gly-Trp-Tyr-Cys-Asp-Asn-Ala-Gly-Ser; and,

(338-348)     Asp-Arg-Gly-Trp-Tyr-Cys-Asp-Asn-Ala-Gly-Ser.

Furthermore, the present invention includes vaccines comprising the above-listed peptides. In addition, the present invention includes a method of immunizing a human against HSRV comprising administration of a vaccine comprising the above-listed peptides. In the method according to the present invention, the vaccine may be administered intranasally, intramuscularly, subcutaneously or sublingually and in a dosage ranging from 0.015 µg to .15 mg per kg patient body weight.

Contemplated equivalents include larger peptides, polypeptides and proteins which comprise such amino acid sequences and function to provide a T-cell proliferative response such as that provided by the peptides listed above. Furthermore, contemplated equivalents include similar sized peptides, larger peptides, polypeptides and proteins that function to provide a similar T-cell proliferative response and which comprise amino acid peptide modelled after the Ffrag peptides and having amino acid insertions and/or deletions and/or conservative substitutions.

Example 1     Production of Ffrag$_{(328-355)}$

The Ffrag$_{(328-355)}$ peptide consists of the amino acid sequence Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala. To prepare the peptide for use in a vaccine, the peptide was synthesized by solid phase methodology on an Applied Biosystems Inc. (ABI) 430A peptide synthesizer using ABI's Small Scale Rapid Cycles (SSRC) on a 0.1 mmole scale. SSRC utilizes abbreviated single couple cycles with standard Boc chemistry. The t-Boc-L-amino acids used (1 mmole) were supplied by ABI with standard side-chain protecting groups. The completed peptide was removed from the supporting PAM (phenylacetamidomethyl) resin, concurrently with the side-chain protecting groups, by a standard HF procedure using appropriate cation scavengers (10% v/v anisole, p-cresol plus p-thiocresol, 1,4-butanedithiol plus anisole or DMS plus anisole) depending on the amino acid sequence of the peptide.

The crude peptide, after HF cleavage, was purified by preparative reverse phase chromatography on a Phenomenex C-18 Column (250 x 22.5 mm) using water acetonitrile gradients, each phase containing 0.1 % TFA. The pure fractions (as determined by analytical HPLC) were pooled, acetonitrile evaporated and the aqueous solution lyophilized. The peptide was analyzed by fast atom bombardment mass spectroscopy and resulting $(M+H)^+$ was compared with the anticipated $(M+H)^+$.

Example 2     Production of Other Ffrag Peptides

Any of the other Ffrag peptides listed in the Detailed Description of the Invention Section of this specification may be produced and purified by one having ordinary skill in the art following the disclosure in Example 1.

Example 3        Vaccine Comprising Ffrag (328-355)

The Ffrag$_{(328-355)}$ peptide can be prepared in vaccine dose form by well-known procedures. The vaccine can be administered sublingually, intramuscularly, subcutaneously or intranasally. For parenteral administration, such as intramuscular injection, the immunogen may be combined with a suitable carrier, for example, it may be administered in water, saline or buffered vehicles with or without various adjuvants or immunomodulating agents such as aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, Corynebacterium parvum (Propionobacterium acnes), Bordetella pertussis, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, NJ).

The proportion of immunogen and adjuvant can be varied over a broad range so long as both are present in effective amounts. For example, aluminum hydroxide can be present in an amount of about 0.5% of the vaccine mixture (Al$_2$0$_3$ basis). On a per dose basis, the concentration of the immunogen can range from about 0.015 µg to about 1.5 mg per kilogram per patient body weight. A preferable dosage range is from about 1.5 µg/kg to about 0.15 mg/kg of patient body weight. A suitable dose size in humans is about 0.1 - 1 ml, preferably about 0.1 ml. Accordingly, a dose for intramuscular injection, for example, would comprise 0.1 ml containing immunogen in admixture with 0.5% aluminum hydroxide.

The vaccine may also be combined with other vaccines for other diseases to produce multivalent vaccines. It may also be combined with other medicaments such as antibiotics.

Example 4        Vaccines Comprising of Ffrag Peptides

Any of the other Ffrag peptides listed in the Detailed Description of the Invention Section of this specification may be used to prepare vaccines by one having ordinary skill in the art following the disclosure in Example 3.

**Claims**

**Claims for the following Contracting States : AT, BE, CH, LI, DE, DK, FR, GB, GR, IT, LU, NL, SE**

1.    A peptide having the formula:

$$R_1\text{-Arg-Gly-Trp-Tyr-Cys-Asp-Asn-Ala-Gly-}R_2$$

wherein $R_1$ is selected from

```
Asp, Thr-Asp, Arg-Thr-Asp, Thr-Arg-Thr-Asp, Leu-Thr-
Arg-Thr-Asp, Cys-Leu-Thr-Arg-Thr-Asp, Ile-Cys-Leu-Thr-
Arg-Thr-Asp, Asn-Ile-Cys-Leu-Thr-Arg-Thr-Asp, Ser-Asn-
Ile-Cys-Leu-Thr-Arg-Thr-Asp, Gly-Ser-Asn-Ile-Cys-Leu-
Thr-Arg-Thr-Asp and Glu-Gly-Ser-Asn-Ile-Cys-Leu-Thr-
Arg-Thr-Asp;
```

and
$R_2$ is selected from

```
Ser, Ser-Val, Ser-Val-Ser, Ser-Val-Ser-Phe, Ser-Val-
Ser-Phe-Phe, Ser-Val-Ser-Phe-Phe-Pro, Ser-Val-Ser-Phe-
Phe-Pro-Gln and Ser-Val-Ser-Phe-Phe-Pro-Gln-Ala.
```

**2.** A peptide according to claim 1, wherein $R_1$ is Glu-Gly-Ser-Asn-Ile-Cys-Leu-Thr-Arg-Thr-Asp.

**3.** A peptide according to claim 2, having the formula:

```
Glu-Gly-Ser-Asn-Ile-Cys-Leu-Thr-Arg-Thr-Asp-Arg-Gly-
Trp-Tyr-Cys-Asp-Asn-Ala-Gly-Ser-Val-Ser-Phe-Phe-Pro-
Gln-Ala.
```

**4.** A peptide according to claim 2, having the formula:

```
Glu-Gly-Ser-Asn-Ile-Cys-Leu-Thr-Arg-Thr-Asp-Arg-Gly-
Trp-Tyr-Cys-Asp-Asn-Ala-Gly-Ser.
```

**5.** A peptide according to claim 1, having the formula:

```
Asp-Arg-Gly-Trp-Tyr-Cys-Asp-Asn-Ala-Gly-Ser-Val-Ser-
Phe-Phe-Pro-Gln-Ala.
```

**6.** A vaccine comprising a peptide according to any preceding claim.

**7.** Use of a peptide according to any of claims 1 to 5, for the manufacture of a medicament for use in immunising a human against HRSV.

**8.** Use according to claim 7, wherein the medicament is adapted for intranasal, intramuscular, subcutaneous or sublingual administration.

**9.** Use according to claim 8, wherein the medicament is adapted for sublingual administration.

**Claims for the following Contracting State : ES**

**1.** A process for preparing a peptide having the formula:

$$R_1-Arg-Gly-Trp-Tyr-Cys-Asp-Asn-Ala-Gly-R_2$$

wherein $R_1$ is selected from

```
Asp, Thr-Asp, Arg-Thr-Asp, Thr-Arg-Thr-Asp, Leu-Thr-
Arg-Thr-Asp, Cys-Leu-Thr-Arg-Thr-Asp, Ile-Cys-Leu-Thr-
Arg-Thr-Asp, Asn-Ile-Cys-Leu-Thr-Arg-Thr-Asp, Ser-Asn-
Ile-Cys-Leu-Thr-Arg-Thr-Asp, Gly-Ser-Asn-Ile-Cys-Leu-
Thr-Arg-Thr-Asp and Glu-Gly-Ser-Asn-Ile-Cys-Leu-Thr-
Arg-Thr-Asp;
```

and
$R_2$ is selected from

```
Ser, Ser-Val, Ser-Val-Ser, Ser-Val-Ser-Phe, Ser-Val-
Ser-Phe-Phe, Ser-Val-Ser-Phe-Phe-Pro, Ser-Val-Ser-Phe-
Phe-Pro-Gln and Ser-Val-Ser-Phe-Phe-Pro-Gln-Ala,
```

which comprises synthesising the peptide by solid phase methodology.

2. A process according to claim 1, wherein $R_1$ is Glu-Gly- Ser-Asn-Ile-Cys-Leu-Thr-Arg-Thr-Asp.

3. A process according to claim 2, having the formula:

```
Glu-Gly-Ser-Asn-Ile-Cys-Leu-Thr-Arg-Thr-Asp-Arg-Gly-
Trp-Tyr-Cys-Asp-Asn-Ala-Gly-Ser-Val-Ser-Phe-Phe-Pro-
Gln-Ala.
```

4. A process according to claim 2, having the formula:

```
Glu-Gly-Ser-Asn-Ile-Cys-Leu-Thr-Arg-Thr-Asp-Arg-Gly-
Trp-Tyr-Cys-Asp-Asn-Ala-Gly-Ser.
```

5. A process according to claim 1, having the formula:

```
Asp-Arg-Gly-Trp-Tyr-Cys-Asp-Asn-Ala-Gly-Ser-Val-Ser-
Phe-Phe-Pro-Gln-Ala.
```

6. A method for making a vaccine, comprising formulating a peptide according to any preceding claim with a carrier.

7. Use of a peptide according to any of claims 1 to 5, for the manufacture of a medicament for use in immunising a human against HRSV.

8. Use according to claim 7, wherein the medicament is adapted for intranasal, intramuscular, subcutaneous or sublingual administration.

9. Use according to claim 8, wherein the medicament is adapted for sublingual administration.

**Patentansprüche**

**Patentansprüche für folgende Vertragsstaaten : AT, BE, CH, DE, DK, FR, GB, GR, IT, LI, LU, NL, SE**

1. Peptid der Formel:

$$R_1\text{-Arg-Gly-Trp-Tyr-Cys-Asp-Asn-Ala-Gly-}R_2,$$

worin bedeuten:
$R_1$

```
Asp, Thr-Asp, Arg-Thr-Asp, Thr-Arg-Thr-Asp, Leu-Thr-
Arg-Thr-Asp, Cys-Leu-Thr-Arg-Thr-Asp, Ile-Cys-Leu-
Thr-Arg-Thr-Asp, Asn-Ile-Cys-Leu-Thr-Arg-Thr-Asp,
Ser-Asn-Ile-Cys-Leu-Thr-Arg-Thr-Asp, Gly-Ser-Asn-
Ile-Cys-Leu-Thr-Arg-Thr-Asp oder Glu-Gly-Ser-Asn-
Ile-Cys-Leu-Thr-Arg-Thr-Asp;
```

und

$R_2$

```
Ser, Ser-Val, Ser-Val-Ser, Ser-Val-Ser-Phe, Ser-Val-
Ser-Phe-Phe, Ser-Val-Ser-Phe-Phe-Pro, Ser-Val-Ser-
Phe-Phe-Pro-Gln oder Ser-Val-Ser-Phe-Phe-Pro-Gln-Ala
```

**2.** Peptid nach Anspruch 1, worin $R_1$ für Glu-Gly-Ser-Asn-Ile-Cys-Leu-Thr-Arg-Thr-Asp steht.

**3.** Peptid nach Anspruch 2 der Formel:

```
Glu-Gly-Ser-Asn-Ile-Cys-Leu-Thr-Arg-Thr-Asp-Arg-Gly-
Trp-Tyr-Cys-Asp-Asn-Ala-Gly-Ser-Val-Ser-Phe-Phe-Pro-
Gln-Ala.
```

**4.** Peptid nach Anspruch 2 der Formel:

```
Glu-Gly-Ser-Asn-Ile-Cys-Leu-Thr-Arg-Thr-Asp-Arg-Gly-
Trp-Tyr-Cys-Asp-Asn-Ala-Gly-Ser.
```

**5.** Peptid nach Anspruch 1 der Formel:

```
Asp-Arg-Gly-Trp-Tyr-Cys-Asp-Asn-Ala-Gly-Ser-Val-Ser-
Phe-Phe-Pro-Gln-Ala.
```

**6.** Impfstoff, umfassend ein Peptid nach einem der vorhergehenden Ansprüche.

**7.** Verwendung eines Peptids nach einem der Ansprüche 1 bis 5 zur Herstellung eines Medikaments zur Verwendung bei der Immunisierung eines Menschen gegen HRSV.

**8.** Verwendung nach Anspruch 7, wobei das Medikament für eine intranasale, intramuskuläre, subkutane oder sublinguale Verabreichung hergerichtet ist.

**9.** Verwendung nach Anspruch 8, wobei das Medikament für eine sublinguale Verabreichung hergerichtet ist.

**Patentansprüche für folgenden Vertragsstaat : ES**

**1.** Verfahren zur Herstellung eines Peptids der Formel:

$$R_1\text{-Arg-Gly-Trp-Tyr-Cys-Asp-Asn-Ala-Gly-}R_2,$$

worin bedeuten:
$R_1$

Asp, Thr-Asp, Arg-Thr-Asp, Thr-Arg-Thr-Asp, Leu-Thr-Arg-Thr-Asp, Cys-Leu-Thr-Arg-Thr-Asp, Ile-Cys-Leu-Thr-Arg-Thr-Asp, Asn-Ile-Cys-Leu-Thr-Arg-Thr-Asp, Ser-Asn-Ile-Cys-Leu-Thr-Arg-Thr-Asp, Gly-Ser-Asn-Ile-Cys-Leu-Thr-Arg-Thr-Asp oder Glu-Gly-Ser-Asn-Ile-Cys-Leu-Thr-Arg-Thr-Asp;

und
$R_2$

Ser, Ser-Val, Ser-Val-Ser, Ser-Val-Ser-Phe, Ser-Val-Ser-Phe-Phe, Ser-Val-Ser-Phe-Phe-Pro, Ser-Val-Ser-Phe-Phe-Pro-Gln oder Ser-Val-Ser-Phe-Phe-Pro-Gln-Ala

durch Synthese des Peptids nach der Festphasenmethode.

2. Verfahren nach Anspruch 1, worin $R_1$ für Glu-Gly-Ser-Asn-Ile-Cys-Leu-Thr-Arg-Thr-Asp steht.

3. Verfahren nach Anspruch 2 der Formel:

Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala.

4. Verfahren nach Anspruch 2 der Formel:

Glu-Gly-Ser-Asn-Ile-Cys-Leu-Thr-Arg-Thr-Asp-Arg-Gly-Trp-Tyr-Cys-Asp-Asn-Ala-Gly-Ser.

5. Verfahren nach Anspruch 1 der Formel:

Asp-Arg-Gly-Trp-Tyr-Cys-Asp-Asn-Ala-Gly-Ser-Val-Ser-Phe-Phe-Pro-Gln-Ala.

6. Verfahren zur Herstellung eines Impfstoffs durch Vereinigen eines Peptids nach einem der vorhergehenden Ansprüche mit einem Träger.

7. Verwendung eines Peptids nach einem der Ansprüche 1 bis 5 zur Herstellung eines Medikaments zur Verwendung bei der Immunisierung eines Menschen gegen HRSV.

8. Verwendung nach Anspruch 7, wobei das Medikament für eine intranasale, intramuskuläre, subkutane oder sublinguale Verabreichung hergerichtet ist.

9. Verwendung nach Anspruch 8, wobei das Medikament für eine sublinguale Verabreichung hergerichtet ist.

**Revendications**

**Revendications pour les Etats contractants suivants : AT, BE, CH, DE, DK, FR, GB, GR, IT, LI, LU, NL, SE**

1.  Peptide répondant à la formule :

    $$R_1\text{-Arg-Gly-Trp-Tyr-Cys-Asp-Asn-Ala-Gly-}R_2$$

    dans laquelle $R_1$ est choisi entre

    Asp, Thr-Asp, Arg-Thr-Asp, Thr-Arg-Thr-Asp, Leu-Thr-Arg-Thr-Asp, Cys-Leu-Thr-Arg-Thr-Asp, Ile-Cys-Leu-Thr-Arg-Thr-Asp, Asn-Ile-Cys-Leu-Thr-Arg-Thr-Asp, Ser-Asn-Ile-Cys-Leu-Thr-Arg-Thr-Asp, Gly-Ser-Asn-Ile-Cys-Leu-Thr-Arg-Thr-Asp et Glu-Gly-Ser-Asn-Ile-Cys-Leu-Thr-Arg-Thr-Asp ;

    et
    $R_2$ est choisi entre

    Ser, Ser-Val, Ser-Val-Ser, Ser-Val-Ser-Phe, Ser-Val-Ser-Phe-Phe, Ser-Val-Ser-Phe-Phe-Pro, Ser-Val-Ser-Phe-Phe-Pro-Glyn et Ser-Val-Ser-Phe-Phe-Pro-Gln-Ala.

2.  Peptide suivant la revendication 1, dans lequel $R_1$ répond à la formule Glu-Gly-Ser-Asn-Ile-Cys-Leu-Thr-Arg-Thr-Asp.

3.  Peptide suivant la revendication 2, répondant à la formule :

    Glu-Gly-Ser-Asn-Ile-Cys-Leu-Thr-Arg-Thr-Asp-Arg-Gly-Trp-Tyr-Cys-Asp-Asn-Ala-Gly-Ser-Val-Ser-Phe-Phe-Pro-Gln-Ala.

4.  Peptide suivant la revendication 2, répondant à la formule :

    Glu-Gly-Ser-Asn-Ile-Cys-Leu-Thr-Arg-Thr-Asp-Arg-Gly-Trp-Tyr-Cys-Asp-Asn-Ala-Gly-Ser.

5.  Peptide suivant la revendication 1, répondant à la formule :

    Asp-Arg-Gly-Trp-Tyr-Cys-Asp-Asn-Ala-Gly-Ser-Val-Ser-Phe-Phe-Pro-Gln-Ala.

6.  Vaccin comprenant un peptide suivant l'une quelconque des revendications précédentes.

7.  Utilisation d'un peptide suivant l'une quelconque des revendications 1 à 5, pour la production d'un médicament destiné à être utilisé dans l'immunisation d'un patient humain contre le virus syncytial respiratoire humain.

8. Utilisation suivant la revendication 7, dans laquelle le médicament est apte à l'administration intra-nasale, intramusculaire, sous-cutanée ou sublinguale.

9. Utilisation suivant la revendication 8, dans laquelle le médicament est apte à l'administration sublinguale.

**Revendications pour l'Etat contractant suivant : ES**

1. Procédé de préparation d'un peptide répondant à la formule :

$$\text{R}_1\text{-Arg-Gly-Trp-Tyr-Cys-Asp-Asn-Ala-Gly-R}_2$$

dans laquelle $\text{R}_1$ est choisi entre

```
Asp, Thr-Asp, Arg-Thr-Asp, Thr-Arg-Thr-Asp, Leu-
Thr-Arg-Thr-Asp, Cys-Leu-Thr-Arg-Thr-Asp, Ile-
Cys-Leu-Thr-Arg-Thr-Asp,    Asn-Ile-Cys-Leu-Thr-
Arg-Thr-Asp,    Ser-Asn-Ile-Cys-Leu-Thr-Arg-Thr-
Asp, Gly-Ser-Asn-Ile-Cys-Leu-Thr-Arg-Thr-Asp et
Glu-Gly-Ser-Asn-Ile-Cys-Leu-Thr-Arg-Thr-Asp ;
```

et
$\text{R}_2$ est choisi entre

```
Ser, Ser-Val, Ser-Val-Ser, Ser-Val-Ser-Phe, Ser-
Val-Ser-Phe-Phe, Ser-Val-Ser-Phe-Phe-Pro, Ser-
Val-Ser-Phe-Phe-Pro-Gln et Ser-Val-Ser-Phe-Phe-
Pro-Gln-Ala,
```

qui comprend la synthèse du peptide par la méthodologie en phase solide.

2. Procédé suivant la revendication 1, dans lequel $\text{R}_1$ répond à la formule Glu-Gly-Ser-Asn-Ile-Cys-Leu-Thr-Arg-Thr-Asp.

3. Procédé suivant la revendication 2, dans lequel le peptide répond à la formule :

```
Glu-Gly-Ser-Asn-Ile-Cys-Leu-Thr-Arg-Thr-Asp-Arg-
Gly-Trp-Tyr-Cys-Asp-Asn-Ala-Gly-Ser-Val-Ser-Phe-
Phe-Pro-Gln-Ala.
```

4. Procédé suivant la revendication 2, dans lequel le peptide répond à la formule :

```
Glu-Gly-Ser-Asn-Ile-Cys-Leu-Thr-Arg-Thr-Asp-Arg-
Gly-Trp-Tyr-Cys-Asp-Asn-Ala-Gly-Ser.
```

5. Procédé suivant la revendication 1, dans lequel le peptide répond à la formule :

18

```
Asp-Arg-Gly-Trp-Tyr-Cys-Asp-Asn-Ala-Gly-Ser-Val-
Ser-Phe-Phe-Pro-Gln-Ala.
```

6. Procédé de production d'un vaccin, comprenant la formulation d'un peptide suivant l'une quelconque des revendications précédentes avec un support.

7. Utilisation d'un peptide suivant l'une quelconque des revendications 1 à 5, pour la production d'un médicament destiné à être utilisé dans l'immunisation d'un patient humain contre le virus syncytial respiratoire humain.

8. Utilisation suivant la revendication 7, dans laquelle le médicament est apte à l'administration intra-nasale, intramusculaire, sous-cutanée ou sublinguale.

9. Utilisation suivant la revendication 8, dans laquelle le médicament est apte à l'administration sublinguale.